# EUROPEAN PATENT SPECIFICATION

(11) **EP 2 226 069 B1**
(45) Date of publication and mention of the grant of the patent: **23.01.2013**
(21) Application number: 08856978.5
(22) Date of filing: 04.12.2008
(51) Int. Cl.: A61K 9/70, A61K 47/10, A61K 47/26, A61K 47/30, A61K 9/00

(54) **EDIBLE FILM**
ESSBARE FOLIE
FILM COMESTIBLE

(30) Priority: 06.12.2007 JP 2007315809
(43) Date of publication of application: 08.09.2010
(73) Proprietor: Lintec Corporation, Tokyo 173-0001 (JP)
(72) Inventor: SUGIURA, Yusaku, Tokyo 173-0001 (JP); KABUTO, Akio, Tokyo 173-0001 (JP); SUZUKI, Eiji, Tokyo 173-0001 (JP)
(74) Representative: Solf, Alexander
(86) International application number: PCT/JP2008/072075
(87) International publication number: WO 2009/072572

(56) References cited:
- EP-A1- 1 391 212
- EP-A1- 1 757 308
- WO-A1-02/087622
- WO-A1-2008/126488
- JP-A- 2004 248 665
- JP-A- 2005 289 867
- JP-A- 2006 160 617
- JP-A- 2007 254 340
- JP-A- 2007 254 341
- US-A- 4 855 142
- DATABASE WPI Week 198620 Thomson Scientific, London, GB; AN 1986-129448 XP002604230 & JP 61 068415 A (TOYOBO KK) 8 April 1986 (1986-04-08)
- DATABASE WPI Week 199401 Thomson Scientific, London, GB; AN 1994-002228 XP002604231 & JP 5 310561 A (LION CORP) 22 November 1993 (1993-11-22)

## Description

### TECHNICAL FIELD

The present invention relates to an edible film.

### RELATED ART

There is known a method of using a film-state edible film for administering functional materials acting on a living body from an oral cavity into a body. Within the oral cavity, the edible film is dissolved by saliva or gelatinized by absorbing water. Therefore, it is relatively easy to swallow the edible film. In addition, since an amount of moisture contained in the edible film can be made small, such an edible film is difficult to deteriorate and decompose. Therefore, it is easy to handle the edible film when producing and storing the same. Furthermore, if a medicine, and a flavor component such as a sweetener, an aroma chemical, a mouth freshener, and the like are contained in an edible film as functional materials, the edible film is capable of exhibiting various kinds of functions.

Among these edible films, a study has been made extensively on a film-state formulation in which a medicine is contained in an edible film (see, e.g., the following Patent Document 1). Such a film-state formulation is dissolved or softened within an oral cavity. Therefore, it is relatively easy for even aged persons or infants to swallow the film-state formulation. In addition, the film-state formulation is dissolved or softened within the oral cavity, which is different from a conventional solid-state formulation (e.g. tablets and capsules). Accordingly, the film-state formulation has a very low risk of getting stuck in a trachea.

However, in a conventional edible film (film-state formulation), there is a case that it adheres to an inside wall of an oral cavity, in particular, a palate due to a physical reason such as a specific shape of the edible film (e.g. a flat shape having a thickness of a few µm to a few mm) or a chemical reason such as a composition of the film when it is taken. If the edible film adheres to the palate, it is difficult to peel off the edible film from the inside wall of the oral cavity. In such a case, there is a problem in that uncomfortable feelings are given by the edible film adhering to the inside wall of the oral cavity. Furthermore, there is another problem in that functional materials contained in the edible film cannot be reliably delivered to intended parts of a body by allowing the edible film to adhere to the palate.

The Patent Document 1 is JP-A 11-116469 as one example of the related arts.

### SUMMARY OF THE INVENTION

It is an object of the present invention to provide an edible film that it is difficult to adhere to an inside wall of an oral cavity, and even if it adheres to the inside wall, it is possible to easily peel off the edible film therefrom.

Such an object is achieved by the present inventions (1) to (11) described below.
(1) An edible film to be ingested from an oral cavity of a living body into a body thereof, the edible film comprised of a laminated body consisting of a plurality of layers, wherein the laminated body has surfaces and the plurality of layers include two surface layers forming of the surfaces of the laminated body, wherein at least one of the two surface layers is disposed as an antiadhesive layer for preventing the edible film from adhering to an inside wall of the oral cavity by dissolving with water.

(2) In the edible film described in the above-mentioned item (1), the antiadhesive layer contains an antiadhesive agent mainly, wherein when an aqueous solution of 5 mass% of the antiadhesive agent is prepared, a viscosity of the aqueous solution at 37°C is 50 mPa·s or less.

(3) In the edible film described in the above-mentioned item (1), the antiadhesive layer contains a water-soluble polymer material.

(4) In the edible film described in the above-mentioned item (1), the antiadhesive layer contains sugars.

(5) In the edible film described in the above-mentioned item (1), the antiadhesive layer is disposed as the two surface layers of the laminated body.

(6) In the edible film described in the above-mentioned item (1), a surface of the antiadhesive layer is formed with irregularities.

(7) In the edible film described in the above-mentioned item (1), the plurality of layers include at least one gel-forming layer swelled and gelatinized by absorbing the water, wherein the at least one gel-forming layer is provided between the two surface layers of the laminated body.

(8) In the edible film described in the above-mentioned item (7), the at least one gel-forming layer contains water absorption promoter for promoting a water absorption of the at least one gel-forming layer.

(9) In the edible film described in the above-mentioned item (8), the water absorption promoter includes glycerin.

(10) In the edible film described in the above-mentioned item (1), the plurality of layers include at least one flavor component-containing layer containing a flavor component, wherein the at least one flavor component-containing layer is provided between the two surface layers of the laminated body.

(11) In the edible film described in the above-mentioned item (7), the plurality of layers include at least one medicine-containing layer containing a medicine, and the at least one medicine-containing layer is provided on the at least one gel-forming layer, wherein the edible film is used as an orally film formulation.

According to the present inventions, it is possible to provide an edible film that it is difficult to adhere to an inside wall of an oral cavity, and even if it adheres to the inside wall, it is possible to easily peel off the edible film therefrom.

### BRIEF DESCRIPTION OF THE DRAWINGS

FIG. 1 is a section view showing an edible film in accordance with a first embodiment of the present invention.
FIG. 2 is a section view showing an edible film in accordance with a second embodiment of the present invention.

### DETAILED DESCRIPTION OF THE PREFERRED EMBODIMENTS

Hereinbelow, the present inventions will be described in detail with reference to their preferred embodiments.

An edible film according to the present invention is a laminated body consisting of a plurality of layers. Antiadhesive layers are provided as outermost surface layers consisting surfaces of the laminated body. For this configuration, it becomes difficult for the edible film to adhere to an inside wall of an oral cavity, in particular, a palate when swallowing the edible film. Even if the edible film adheres to the inside wall, it becomes easy to peel off it. It is preferred that the antiadhesive layers are provided as two outermost surface layers of the laminated body constituting the edible film. This reason is as follows; Even if the edible film is swallowed so as to face an either surface of surfaces of the two outermost surface layers of the laminated body to the palate, it becomes difficult for the edible film to adhere to the palate. Even if the edible film adheres to the palate, it is possible to easily peel off it therefrom.

Hereinbelow, as one example of the edible film according to the present invention, a description will be made on a case of an edible film used as an orally film preparation in which a medicine-containing layer containing a medicine is provided.

Hereinbelow, embodiments of the present invention will be described with reference to the accompanying drawings.

### <First Embodiment>

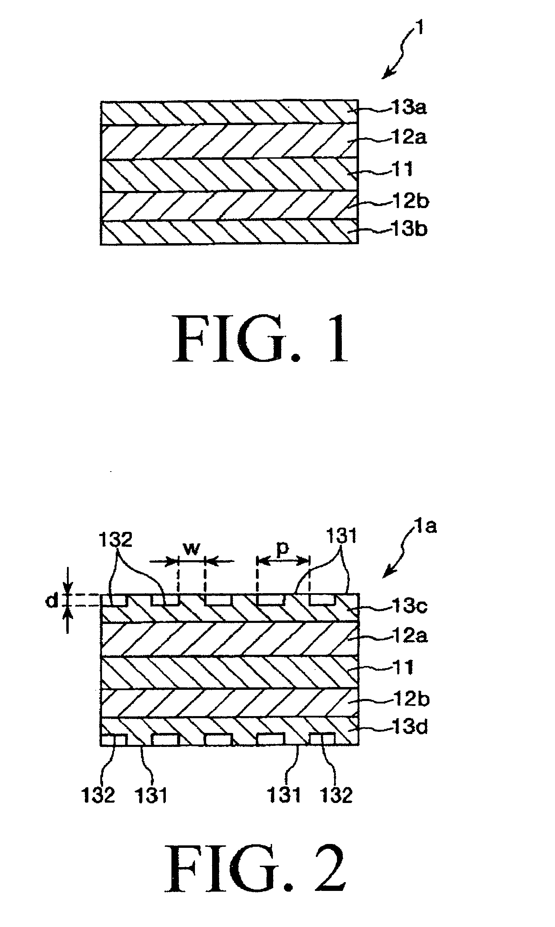

FIG. 1 is a section view showing an edible film in accordance with a first embodiment. In the following description, the upper side in FIG. 1 will be referred to as "upper" and the lower side thereof will be referred to as "lower" for convenience of explanation.

As shown in FIG. 1, the edible film 1 according to the first embodiment is configured as a laminated body. Such a laminated body includes a medicine-containing layer 11 which contains a medicine, a first gel-forming layer 12a laminated on an upper surface of the medicine-containing layer 11, a second gel-forming layer 12b laminated on an lower surface of the medicine-containing layer 11, a first antiadhesive layer 13a laminated on an upper surface of the first gel-forming layer 12a, and a second antiadhesive layer 13b laminated on an lower surface of the second gel-forming layer 12b. The first and second antiadhesive layers 13a and 13b are provided as outermost surface layers constituting surfaces of the edible film 1. Furthermore, each of an upper surface of the first antiadhesive layer 13a and a lower surface of the second antiadhesive layer 13b constitutes an outer surface of the edible film 1. These surfaces are flat.

Since the edible film 1 does not need moisture to store it, it is possible to reduce a moisture content in the film edible 1 when storing the edible film 1 (unusing). Therefore, it is possible to enhance stability of the medicine (especially, an easily-hydrolysable medicine) contained in the medicine-containing layer 11. Moreover, an orally film formulation using the edible film 1 is easy to handle, and can assist in reducing a packing cost of the orally film formulation.

Hereinbelow, the respective layers constituting the edible film 1 will be described in more detail.

### <Medicine-containing Layer>

The medicine-containing layer 11 is a layer containing the medicine to be administered into a living body.

The medicine contained in the medicine-containing layer 11 is a medicine to be administered to a patient. Such a medicine is not limited to a specific one but may be any orally-administrable medicine. Examples of the orally-administrable medicine include: medicines acting on a central nerve, including a hypnotic medicine such as amobarbital, estazoram, triazolam, nitrazepam, pentobarbital or the like, a psychotropic medicine such as amitriptyline hydrochloride, imipramine hydrochloride, oxazolam, chlordiazepoxide, chlorpromazine, diazepam, sulpiride, haloperidol or the like, an antiparkinson medicine such as trihexyphenidyl, levodopa or the like, an analgesic medicine and an anti-inflammatory medicine such as aspirin, isopropylantipyrine, indometacin, diclofenac sodium, mefenamic acid, streptokinase, streptodornase, serrapeptase, pronase or the like and a central nervous metabolic activation medicine such as APT, vinpocetine or the like; medicines acting on a respiratory organ, including an expectorant medicine such as carbocysteine, bromhexine hydrochloride or the like and an antiasthmatic medicine such as azelastine hydrochloride, oxatomide, theophylline, terbutaline sulfate, tranilast, procaterol hydrochloride, ketotifen fumarate or the like; medicines acting on a circulatory system, including a cardiac stimulant such as aminophylline, digitoxin, digoxin or the like, an antiarrhythmic medicine such as ajmaline, disopyramide, procainamide hydrochloride, mexiletine hydrochloride or the like, an antianginal medicine such as amyl nitrite, alprenolol hydrochloride, isosorbide dinitrate, nicorandil, oxyfedrine, dipyridamole, dilazep hydrochloride, diltiazem hydrochloride, nitroglycerin, nifedipine, verapamil hydrochloride or the like, a peripheral vasodilator such as kallidinogenase or the like, an antihypertensive medicine such as atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine or the like and an antiarteriosclerotic medicine such as clofibrat, dextran sulfate, nicomol, niceritrol or the like; blood and hematological medicines, including a hemostatic medicine such as carbazochrome sodium sulfonate, tranexamic acid or the like, an antithrombogenic medicine such as ticlopidine hydrochloride, warfarin potassium or the like and an anemia medicine such as ferric sulfate or the like; medicines acting on a gastrointestinal system, including an antiulcer medicine such as azulene, aldioxa, cimetidine, ranitidine hydrochloride, famotidine, teprenone, rebamipide or the like, an antiemetic medicine such as domperidone, metoclopramide or the like, a cathartic medicine such as sennoside, digestive enzyme preparations, and a therapeutic medicine for liver diseases such as glycyrrhizin, liver extract preparations or the like; medicines acting on a metabolic disease, including an antidiabetic medicine such as glibenclamide, chlorpropamide, tolbutamide or the like and an antipodagric medicine such as allopurinol, colchicines or the like; medicines for an ophthalmic field, including acetazolamide; medicines for an otological field, including an anti-vertigo medicine such as difenidol hydrochloride, betahistine mesylate or the like; chemotherapeutic medicines and antibiotic medicines including isoniazid, ethambutol hydrochloride, ofloxacin, erythromycin stearate, cefaclor, norfloxacin, fosfomycin calcium, minocycline hydrochloride, rifampicin, rokitamycin or the like; antineoplastic medicines including cyclophosphamide, tegafur or the like; immunosuppressive medicines including azathioprine; hormones and endocrine medicines including progestational hormone, salivary hormone, thiamazole, prednisolone, betamethasone, liothyronine, levothyroxine or the like; and physiologically active substances (autacoids) including an antihistamine medicine such as diphenhydramine hydrochloride, clemastine fumarate, D-chlorpheniramine maleate or the like and a vitamin such as alfacalcidol, cobamamide, tocopherol nicotinate, mecobalamin or the like. One or more of these medicines may be used independently or in combination according to the purposes of treatment and prevention of a condition.

Furthermore, various kinds of medicines including a medicine administered in a small quantity and a medicine administered in a large quantity can be contained in the medicine-containing layer 11. In this regard, the medicine administered in a small quantity means a medicine whose one-time dosage amount is 1 mg or less, while the medicine administered in a large quantity means a medicine whose one-time dosage amount is 300 mg or more.

A content of the medicine in the medicine-containing layer 11 is not particularly limited and may be suitably adjusted depending on a kind of medicine and the volume of the medicine-containing layer 11. The content of the medicine is preferably in the range of 0.01 to 70 mass%, more preferably in the range of 0.01 to 40 mass% and even more preferably in the range of 0.01 to 35 mass%. This makes it possible to have a sufficiently quantity of the medicine contained in the edible film 1 while enhancing physical strength of the edible film 1.

Furthermore, the edible film 1 exhibits great enough physical strength even when a relatively large quantity of the medicine as described above is contained in the medicine-containing layer 11 or when an insoluble to water and bulky medicine having a tendency to reduce the physical strength of the medicine-containing layer 11 is contained in the medicine-containing layer 11. Presumably, this is because the gel-forming layers 12a and 12b impart great enough physical strength to the edible film 1 by providing the gel-forming layer 12a provided on the upper surface of the medicine-containing layer 11 and the gel-forming layer 12b provided on the lower surface of the medicine-containing layer 11 in the edible film 1.

The medicine-containing layer 11 may include a base (namely, a base agent for the medicine-containing layer) which serves to keep the administered medicine in a desired state in the medicine-containing layer 11 and to adjust the shape and the physical strength of the medicine-containing layer 11. Examples of the base used in the medicine-containing layer 11 include, but are not limited to: cellulose such as crystalline cellulose, carboxymethyl cellulose, hydroxypropyl cellulose, hydroxypropyl methyl cellulose, methyl cellulose, ethyl cellulose, acetyl cellulose, cellulose acetate phthalate, hydroxypropylmethyl cellulose phthalate, hydroxypropylmethyl cellulose acetate sucinate and carboxymethylethyl cellulose; derivatives of cellulose or pharmaceutically acceptable salts of cellulose (e.g., sodium salt); starch such as a-starch, oxidized starch, carboxymethyl starch sodium, hydroxypropyl starch, dextrin and dextran; derivatives of starch; sugars such as saccharose, maltose, lactose, glucose, fructose, pullulan, xanthane gum, cyclodextrin, xylitol, mannitol and sorbitol; acrylate derivatives such as a dimethylaminoethyl methacrylate-methacrylic acid copolymer, a methacrylic acid-ethylacrylate copolymer, a methacrylic acid-methylmethacrylate copolymer, an ethylmethacrylate-chlorotrimethylammonium methacrylic acid copolymer, a dimethylaminoethyl methacrylate-chloromethyl methacrylate copolymer and a methacrylic acid-chloroethyl acrylate copolymer; Sellac; polyvinylacetal diethylamino acetate; polyvinyl acetate; polyvinyl alcohol; polyvinyl pyrolidone; a vinylacetate-vinylpyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearic acid; magnesium aluminometasilicate; magnesium silicate; and silicic anhydride. One or more of these bases may be used independently or in combination according to the purposes of including the base into the medicine-containing layer 11.

A content of the base in the medicine-containing layer 11 is not particularly limited, but may be preferably in the range of 30 to 99.99 mass%, more preferably in the range of 60 to 99.99 mass% and even more preferably in the range of 65 to 99.99 mass%. This makes it possible to sufficiently enhance the physical strength of the medicine-containing layer 11 with ease while allowing a sufficiently quantity of the medicine to be contained in the medicine-containing layer 11.

A thickness of the medicine-containing layer 11 can be suitably adjusted within a range permitting an oral administration of the edible film 1. The thickness of the medicine-containing layer 11 is not particularly limited, but may be preferably in the range of 0.5 to 1000 µm and more preferably in the range of 10 to 500 µm. This makes it possible to sharply reduce variations in the medicine content and the thickness which would occur in respective portions of the medicine-containing layer 11. In addition, this makes it possible to sufficiently increase overall softness of the edible film 1 and to greatly enhance ease of swallowing the edible film 1.

### <Gel-forming Layer>

The first and second gel-forming layers 12a and 12b are layers that can be swelled and gelatinized by absorbing water. The first gel-forming layer 12a is provided on the upper surface of the medicine-containing layer 11. The second gel-forming layer 12b is provided on the lower surface of the medicine-containing layer 11.

Hereinafter, only the first gel-forming layer 12a will be representatively described below, because the first and second gel-forming layers 12a and 12b have substantially the same configuration.

As described above, the first gel-forming layer 12a can be swelled and gelatinized within the oral cavity of a patient by water contained in saliva etc. This makes it possible to change a state of the edible film 1 to a state of it having a size, a shape, elastic force, a viscosity and the like of swallowing with ease. Accordingly, the patient can easily swallow the edible film 1. Furthermore, the edible film 1 has a low risk that it is likely to get stuck in a trachea of the patient when swallowing the edible film 1. Therefore, in the even case where the patient is aged persons or infants, it is possible to swallow the edible film 1 safely and easily.

Furthermore, the first gel-forming layer 12a can prevent the medicine contained in the medicine-containing la yer 11 from being dissolved into the oral cavity. This make s it possible to mask a taste (e.g. bitter taste, astringent taste and numbness) or an offensive odor of the medicine wh ich would be generated by dissolving the medicine.

The first gel-forming layer 12a contains a gel-forming agent that can be swelled and gelatinized by absorbing water. The first gel-forming layer 12a containing such a gel-forming agent can form a gel by easily and rapidly absorbing the water existing around the edible film 1 within the oral cavity.

Examples of the gel-forming agent include, but are not particularly limited to: a carboxy vinyl polymer; starch and its derivatives; an ager; algin acid; arabinogalactan; galactomannan; cellulose and its derivatives; carragheen; dextran; tragacanth; gelatin; pectin; hyaluronic acid; gellan gum; collagen; casein; xanthane gum; and the like. One or more of these substances can be used independently or in combination.

Among these substances, it is preferred that the gel-forming agent includes the carboxyl vinyl polymer. The carboxyl vinyl polymer can rapidly absorb the water and can form the gel in a rapid manner. In addition, the carboxyl vinyl polymer is a component relatively hard to dissolve after formation of the gel. Therefore, in the case where the carboxyl vinyl polymer is used as the gel-forming agent, the first gel-forming layer 12a is reliably kept in a gelatinized shape within the oral cavity even after the formation of the gel. Furthermore, in the case where a polyacrylic acid in the carboxyl vinyl polymer is used, the effects as described above are exhibited more conspicuously.

In such a case, a viscosity at 20°C of an aqueous solution of 0.2 mass% of the carboxyl vinyl polymer is preferably in the range of 1500 to 50000 mPa·s and more preferably in the range of 10000 to 20000 mPa·s. This enables the gel-forming agent contained in the first gel-forming layer 12a to rapidly absorb the water and to form the gel in the rapid manner. The gel-forming layer 12a is reliably kept in the gelatinized shape.

In the case where the carboxyl vinyl polymer is used as the gel-forming agent, it may be possible to crosslink the carboxyl vinyl polymer through the use of a cross-linking agent. This ensures that the gelatinized first gel-forming layer 12a is surely prevented from dissolution.

The cross-linking can be performed by the cross-linking agent that varies with a kind of molecules to be cross-linked. In the case where the polyacrylic acid is used as the carboxy vinyl polymer, as the cross-linking agent for cross-linking the polyacrylic acid, e.g., a polyvalent metal compound can be used. The polyvalent metal compound cross-linkes the polyacrylic acid as follows; When the gel-forming agent contained in the first gel-forming layer 12a, that is, the polyacrylic acid is swelled and gelatinized within the oral cavity of the patient by the water contained in saliva etc., the polyvalent metal compound is ionized to thereby generate a polyvalent metal ion. Then, the polyvalent metal ion cross-links the polyacrylic acid contained in the first gel-forming layer 12a. Accordingly, even if the sufficiently cross-linked gel-forming agent is not contained in the first gel-forming layer 12a preliminarily, a gel having great enough strength is formed in the first gel-forming layer 12a.

Examples of the polyvalent metal compound include, but are not limited to, calcium chloride, magnesium chloride, aluminum chloride, aluminum sulfate, aluminum potassium sulfate, ferric chloride alum, ammonium alum, ferric sulfate, aluminum hydroxide, aluminum silicate, aluminum phosphate, iron citrate, magnesium oxide, calcium oxide, zinc oxide and zinc sulfate. One or more of these compounds can be used independently or in combination. Use of a trivalent metal compound among these compounds makes it possible to surely prevent dissolution of the polyacrylic acid, while increasing a cross-linking degree of the polyacrylic acid and enhancing physical strength of the first gel-forming layer 12a.

A content of the gel-forming agent in the first gel-forming layer 12a is preferably in the range of 5 to 90 mass% and more preferably in the range of 15 to 70 mass%, although it can be suitably adjusted depending on a kind of gel-forming agent or other factors. This enables the first gel-forming layer 12a to rapidly absorb water. Furthermore, the gel-forming agent is reliably prevented from being dissolved within the oral cavity after gelatinization of the gel-forming agent contained in the first gel-forming layer 12a.

In the case where the cross-linking agent is contained in the first gel-forming layer 12a, a content of the cross-linking agent in the first gel-forming layer 12a is preferably in the range of 0.1 to 2.5 mass% and more preferably in the range of 0.5 to 1.2 mass%. This makes it possible to surely prevent dissolution of the gel-forming agent contained in the first gel-forming layer 12a while easily keeping the first gel-forming layer 12a in the gelatinized shape after the gel-forming agent is gelatinized. In addition, it is possible to reduce a viscosity of a coating solution used as a raw material of the first gel-forming layer 12a in the below-mentioned process of producing the edible film 1, which makes it possible to efficiently form the first gel-forming layer 12a.

The first gel-forming layer 12a may contain a base (namely, a gel-forming base agent) which is a component contributing to stabilization of the shape of the first gel-forming layer 12a. In other words, the base imparts a suitable degree of flexibility to the first gel-forming layer 12a before the gel-forming agent is swelled by water. Accordingly, the inclusion of the base to the first gel-forming layer 12a makes it possible to prevent the edible film 1 from being cracked or damaged by an external force or other causes. After the gel-forming agent contained in the first gel-forming layer 12a has absorbed the water, the base serves to reliably keep the first gel-forming layer 12a in the gelatinized shape, which prevents the gel from flowing out the first gel-forming layer 12a.

Examples of the base used in the first gel-forming layer 12a include, but are not limited to, polyvinyl alcohol, polyvinyl pyrolidone, polyvinyl acetate, polyvinylphthalate acetate, hydroxyalkyl cellulose (e.g., hydroxypropyl cellulose, hydroxypropylmethyl cellulose, hydroxymethyl cellulose or hydroxyethyl cellulose), alkyl cellulose (e.g., methyl cellulose or ethyl cellulose), carboxyalkyl cellulose (e.g. carboxymethyl cellulose), (metha)acrylic acid and its ester, xanthan gum, carrageenan, alginic acid and the like. One or more of these compounds can be used independently or in combination.

In the case where the base is contained in the first gel-forming layer 12a, a content of the base in the first gel-forming layer 12a is preferably in the range of 20 to 85 mass% and more preferably in the range of 30 to 80 mass%.

It is preferred that the base contained in the first gel-forming layer 12a is water-soluble. If the base is water-soluble, it becomes easy for water to get into the first gel-forming layer 12a, thereby enabling the gel-forming agent contained in the first gel-forming layer 12a to be rapidly swelled and gelatinized within the oral cavity.

Examples of the water-soluble base include: polyvinyl alcohol; hydroxyalkyl cellulose such as hydroxypropyl cellulose, hydroxypropylmethyl cellulose, methyl cellulose or the like; polyvinyl pyrolidone; xanthan gum; carrageenan; alginic acid; and the like. One or more among these compounds can be used independently or in combination.

In particular, in the case where polyvinyl alcohol is included in the base contained in the first gel-forming layer 12a, even if the medicine contained in the medicine-containing layer 11 flows out from the medicine-containing layer 11 into the oral cavity, polyvinyl alcohol can mask the taste or order of the medicine contained in the medicine-containing layer 11. That is to say, polyvinyl alcohol can also serve as a masking agent to be described later.

The first gel-forming layer 12a may contain a water absorption promoter for promoting water absorption of the first gel-forming layer 12a. If the first gel-forming layer 12a contains the water absorption promoter, it becomes possible to sufficiently increase the water absorption speed of the first gel-forming layer 12a within the oral cavity.

As the water absorption promoter, it is possible to use, e.g., a component having relatively high water-solubility. This component having relatively high water-solubility is dissolved in water and therefore can transport water into the first gel-forming layer 12a.

When an aqueous solution of the water absorption promoter of 5 mass% is prepared, a viscosity at 37°C of the aqueous solution is preferably in the range of 0.3 to 5.0 mPa·s, more preferably in the range of 0.5 to 3.5 mPa·s and even more preferably in the range of 0.6 to 1.8 mPa·s. As an indicator of water solubility of the water absorption promoter, it is possible to use, e.g., the viscosity of the aqueous solution in which the water absorption promoter is dissolved. It is possible to think that the water solubility of the water absorption promoter is improved as the viscosity of the aqueous solution grows low. However, if the viscosity of the aqueous solution of the water absorption promoter of 5 mass% falls within the range noted above, the water absorption promoter has suitably a high degree of the water solubility within the oral cavity. This makes it possible to suitably increase the water absorption speed of the first gel-forming layer 12a containing the gel-forming agent. Furthermore, this also makes it possible to surely prevent the water absorption promoter from being suddenly dissolved and dispersed into the saliva.

Examples of the water absorption promoter include, but are not limited to: glycols such as propylene glycol, polyethylene glycol, polypropylene glycol, polyoxyl stearate, polyoxyethylene polyoxypropylene glycol, polyoxyethylene-cured castor oil and the like; glycerin; and sugars such as erythritol, sorbitol, xylitol, mannitol, inositol, maltitol, lactitol, glucose, xylose, mannose, fructose, galactose, sucrose, fructose, saccharose and the like. One or more of these compounds can be used independently or in combination.

It is preferred that the water absorption promoter contains glycerin among the compounds listed above. Glycerin is a component that has increased capability to promote the water absorption of the first gel-forming layer 12a and has a function of being capable of imparting softness to the first gel-forming layer 12a. Therefore, the edible film 1 has a suitable degree of flexibility until it is administered to a patient, which means that the edible film 1 is hardly broken or damaged by an external force. After the edible film 1 is administered to the patient, the first gel-forming layer 12a becomes soft within the oral cavity while keeping its shape unchanged due to functions of glycerin described above. Thus, the edible film 1 becomes easy to swallow and therefore even if the edible film 1 adheres to the inside wall of the oral cavity once, the edible film 1 becomes particularly easy to peel off. Furthermore, even if the medicine contained in the medicine-containing layer 11 flows out from the medicine-containing layer 11 to the oral cavity, glycerin is a component of be capable of masking the bitter taste or odor of the medicine contained in the medicine-containing layer 11 within the oral cavity due to the sweet taste of glycerin.

It the case where the water absorption promoter contains the sugars among the compounds described above, it becomes possible to attain the following advantageous effects. More specifically, the sugars can serve as a masking agent to be described later, because they taste sweet and have increased capability to promote the water absorption of the first gel-forming layer 12a. Furthermore, the sweet taste of the sugars felt within the oral cavity by a patient helps accelerate secretion of the saliva. As a result, the edible film 1 shows increased swallowability. Furthermore, in the case where the water absorption promoter contains the sugars and glycerin, the sugars and glycerin are similar in a chemical structure thereof, and therefore they have an extremely high affinity with respect to each other. Accordingly, the sugars are capable of reliably holding glycerin in the first gel-forming layer 12a and surely preventing glycerin from flowing out (bleeding) from the edible film 1 when storing the edible film 1.

In the case where the water absorption promoter contains glycols among the compounds described above, it becomes possible to attain the following advantageous effects. Since glycols show a good affinity to water and have a chain-like structure in a chemical structure thereof, glycols are a component that can be easily intertwined to molecules of glycols in themselves or other molecules than glycols contained in the first gel-forming layer 12a. Therefore, glycols are linked to other molecules in the first gel-forming layer 12a, thus maintaining the shape of the first gel-forming layer 12a. This ensures that the gel-forming agent contained in the first gel-forming layer 12a is gelatinized with great ease while maintaining a shape of the first gel-forming layer 12a. As a result, the edible film 1 shows especially high swallowability.

A content of the water absorption promoter in the first gel-forming layer 12a is preferably in the range of 1 to 20 mass% and more preferably in the range of 3 to 17 mass%. This makes it possible to greatly increase the water absorption speed of the first gel-forming layer 12a, while keeping the first gel-forming layer 12a in a desired gel shape within the oral cavity.

The first gel-forming layer 12a may contain a plasticizer. By containing the plasticizer, a proper degree of the softness is imparted to the first gel-forming layer 12a. Examples of the plasticizer include glycerin triacetate, diethyl phthalate, triethyl citrate and laurylic acid, one or more of which can be used independently or in combination.

The first gel-forming layer 12a may contain a masking agent capable of masking the taste or odor of the medicine contained in the medicine-containing layer 11. By containing the masking agent into the first gel-forming layer 12a, it is possible to enhance the effect of masking the taste or odor of the medicine contained in the medicine-containing layer 11 (what is called a masking effect), even if the medicine contained in the medicine-containing layer 11 flows out from the medicine-containing layer 11 to the oral cavity. Examples of the masking agent include: acidic-taste imparting agents such as citric acid, tartaric acid, fumaric acid and the like; sweetening agents such as saccharin, glycyrrhizinic acid, aspartame, stevioside, acesulfame potassium, sugars and the like; mouth fresheners such as menthol, mentha oil, peppermint, spearmint and the like; and natural or synthetic perfumes. One or more among these compounds can be used independently or in combination. The afore-mentioned sugars as the water absorption promoter have a sweet taste and can serve as the masking agent.

The first gel-forming layer 12a may contain other components than mentioned above. For example, the first gel-forming layer 12a may contain: antiseptic agents such as methyl hydroxybezoate, propyl hydroxybezoate and the like; and coloring agents such as edible lake pigment and the like.

A thickness of the first gel-forming layer 12a is preferably in the range of 5 to 1000 µm and more preferably in the range of 10 to 500 µm, although it may be suitably adjusted within an orally-administrable range. If the thickness of the first gel-forming layer 12a is smaller than 5 µm, the first gel-forming layer 12a is gelatinized insufficiently and the effect of masking the taste or odor of the medicine contained in the medicine-containing layer 11 by the first gel-forming layer 12a becomes insufficient. On the other hand, if the thickness of the first gel-forming layer 12a is greater than 1000 µm, the gel-forming agent contained in the first gel-forming layer 12a cannot be sufficiently swelled and gelatinized only with the saliva when the edible film 1 is administered into the oral cavity of the patient. As a result, it becomes difficult to swallow the edible film 1.

### <Antiadhesive Layer>

The first antiadhesive layer 13a is laminated on the upper surface of the first gel-forming layer 12a. Such a first antiadhesive layer 13a is provided as a surface layer constituting one surface of the edible film 1 (laminated body). On the other hand, the second antiadhesive layer 13b is laminated on the lower surface of the second gel-forming layer 12b. Such a second antiadhesive layer 13b is provided as a surface layer constituting the other surface of the edible film 1 (laminated body).

Furthermore, the first and second antiadhesive layers 13a and 13b are rapidly dissolved by water such as saliva within the oral cavity and have a function of preventing the edible film 1 from adhering to the inside wall in the oral cavity.

Meanwhile, there is a case that a conventional edible film adheres to an inside wall of an oral cavity, in particular, a palate, when it is taken. If the edible film adheres to the inside wall, it is difficult to take the edible film off the inside wall of the oral cavity. In such a case, there is a case that uncomfortable feelings are brought by allowing the edible film administered in the oral cavity to adhere to the inside wall of the oral cavity. Furthermore, there is a problem in that medicines contained in the edible film cannot be reliably transferred to intended parts of a body. In particular, it is difficult to swallow the edible film by allowing it to adhere to the inside wall of the oral cavity. Thereafter, the edible film is dissolved in the oral cavity with ease, so that the medicines contained in the edible film are likely to flow out from it. Therefore, there is a problem in that it is impossible to apply or use a medicine which tends to give unpleasant tastes (e.g., a bitter taste, an astringent taste and numbness) or offensive odors to the edible film.

In contrast, the edible film 1 according to the present invention has the first and second antiadhesive layers 13a and 13b as the outermost surface layers of the laminated body. The first and second antiadhesive layers 13a and 13b are rapidly dissolved by water such as saliva within the oral cavity and have a function of preventing the edible film 1 from adhering to the inside wall of the oral cavity. In other words, when the edible film 1 is taken in the oral cavity, surface parts of the first and second antiadhesive layers 13a and 13b are quickly dissolved by the saliva, thereby rapidly forming water-state films between the first and second antiadhesive layers 13a and 13b and the inside wall of the oral cavity, respectively. As a result, it becomes easy for the first and second antiadhesive layers 13a and 13b of the edible film 1 to slide with respect to the inside wall. Therefore, the edible film 1 is prevented from being in contact with the inside wall of the oral cavity, so that it becomes difficult to adhere to the inside wall of the oral cavity. Furthermore, even if parts of the first and second antiadhesive layers 13a and 13b of the edible film 1 adhere to the inside wall, it becomes easy for the first and second antiadhesive layers 13a and 13b of the edible film 1 to peel off from the inside wall of the oral cavity. This makes it possible to prevent uncomfortable feelings from being brought by allowing the first and second antiadhesive layers 13a and 13b of the edible film 1 to adhere to the inside wall of the oral cavity, so that it becomes easy to swallow the edible film 1. Furthermore, it is possible to reliably transfer the medicine contained in the medicine-containing layer 11 to the intended parts of the body. In addition, it becomes possible to apply or use a medicine which tends to give unpleasant tastes (e.g., a bitter taste, an astringent taste and numbness) or offensive odors to the edible film 1.

Particularly, in the present embodiment, the edible film 1 has the first and second gel-forming layers 12a and 12b. Therefore, the edible film 1 becomes soft in the oral cavity. This makes it possible for the first and second antiadhesive layers 13a and 13b of the edible film 1 to more easily peel off from the inside wall of the oral cavity by deformation of the edible film 1 with week power, even if parts of the first and second antiadhesive layers 13a and 13b of the edible film 1 adhere to the inside wall of the oral cavity.

In addition to that, since the first antiadhesive layer 13a prevent the medicine contained in the medicine-containing layer 11 from being dissolved within the oral cavity. Even if the medicine flows out into the oral cavity, it is possible to mask tastes (e.g., a bitter taste, an astringent taste and numbness) or odor of the medicine.

Hereinafter, since the first and second antiadhesive layers 13a and 13b are substantially identical with each other in configurations thereof, a description will be made on the first antiadhesive layer 13a as representative.

The first antiadhesive layer 13a is rapidly dissolved by water contained in saliva etc. within the oral cavity, and is mainly constituted of an antiadhesive agent which is capable of forming a water-state film around the edible film 1. By including such an antiadhesive agent, it becomes difficult for the edible film 1 to be in contact with the inside wall of the oral cavity, so that it becomes easy to reliably slide with respect to the inside wall of the oral cavity. AS a result, it becomes difficult for the edible film 1 to adhere to the inside wall of the oral cavity, thereby exhibiting superior swallowability.

Furthermore, in the antiadhesive agent as described above, when an aqueous solution of the antiadhesive agent of 5 mass% is prepared, a viscosity at 37°C of the aqueous solution is preferably 50 mPa·s or less, more preferably 40 mPa·s or less and even more preferably 30 mPa·s or less. As an indicator of adhesive property of the first antiadhesive layer 13a with respect to the inside wall of the oral cavity, it is possible to use the viscosity of the aqueous solution in which the antiadhesive agent constituting the first antiadhesive layer 13a is dissolved. The antiadhesive agent constituting the first antiadhesive layer 13a is quickly dissolved in water in the oral cavity and therefore an aqueous-solution-state film having a low viscosity is formed around the edible film 1 with ease as the viscosity of the aqueous solution grows low. This makes it possible for the edible film 1 to easily slide with respect to the inside wall of the oral cavity, which makes it difficult to adhere to the inside wall. In view of the above, it becomes more difficult for the edible film 1 to adhere to the inside wall of the oral cavity, thereby exhibiting superior swallowability. This is because the first antiadhesive layer 13a mainly includes the antiadhesive agent which is contained in the aqueous solution thereof having sufficiently the low viscosity when the antiadhesive agent is dissolved in water with the concentration as described above.

In the first antiadhesive layer 13a, the phrase "mainly includes the antiadhesive agent" means that a concentration of the antiadhesive agent contained in the first antiadhesive layer 13a is 50 mass% or higher. A content of the antiadhesive agent in the first antiadhesive layer 13a is preferably 50 mass% or higher, more preferably 70 mass% or higher and even more preferably 90 mass% or higher. This makes it possible to conspicuously obtain the effects as described above.

The antiadhesive agent is not particularly limited as long as when the aqueous solution of the antiadhesive agent of 5 mass% is prepared, the viscosity characteristics as described above, that is, the viscosity at 37°C of the aqueous solution falls within the range as described above. Examples of the antiadhesive agent include; a water-soluble polymer material such as hydroxyalkyl cellulose, polyethylene glycol, polypropylene glycol, polyvinyl alcohol, polyoxyl stearate, polyoxyethylene polyoxypropylene glycol, polyoxyethylene-cured castor oil, gum arabic, gelatin and the like; sugars such as erythritol, sorbitol, xylitol, mannitol, inositol, maltitol, lactitol, glucose, xylose, mannose, fructose, galactose, sucrose, fructose, saccharose and the like; propylene glycol; glycerin; and the like. One or more of these compounds can be used independently or in combination. Furthermore, examples of the hydroxyalkyl cellulose include hydroxypropyl cellulose, hydroxypropylmethyl cellulose, methyl cellulose and the like.

Among these compounds, it is preferred that the first antiadhesive layer 13a includes the water-soluble polymer material as the antiadhesive agent. The water-soluble polymer material can be reliably dissolved in water and has a molecular chain having the appropriate length in a chemical structure thereof. Therefore, the molecular chain of the water-soluble polymer material in itself can be intertwined. Furthermore, in the case where the first antiadhesive layer 13a includes a plurality of kinds of water-soluble polymer materials having different solubilities, the molecular chains of the water-soluble polymer materials having the different solubilities can be appropriately intertwined to each other. Therefore, when the water-soluble polymer materials dissolve from the first antiadhesive layer 13a, it is possible to reliably allow the water-soluble polymer materials to unevenly exist around the edible film 1 in a state of an aqueous solution. For this reason, the edible film 1 is reliably prevented from adhering to the inside wall of the oral cavity for a long period of time. Furthermore, the water-soluble polymer material as described above can also serve as a base of the first antiadhesive layer 13a, which is possible to produce the edible film 1 with ease. In addition, it is possible to reliably exhibit superior durability when storing the produced edible film 1. In particularly, among the water-soluble polymer materials, in the case where at least one of polyethylene glycol and polyvinyl alcohol is used, it is possible to conspicuously exhibit the effects as described above.

A mass-average molecular weight of the water-soluble polymer material as described above is preferably in the range of 5000 to 150000 and more preferably in the range of 10000 to 100000. This makes it possible for the water-soluble polymer material to sufficiently improve solubility with respect to water. After the water-soluble polymer material is dissolved, it is possible to reliably allow the water-soluble polymer material to unevenly exist around the edible film 1 in a state of an aqueous solution. Therefore, the edible film 1 is reliably prevented from reliably adhering to the inside wall of the oral cavity for a longer period of time.

Further, among the compounds of the antiadhesive agent described above, it is preferred that the sugars are included in the first antiadhesive layer 13a as the antiadhesive agent. The sugars are capable of serving as a masking agent to mask tastes or odor of the medicine. Furthermore, the sugars are components for accelerating secretion of the saliva in the oral cavity. Since the sugars have superior solubility with respect to water, the sugars not only serve as the antiadhesive agent but also have functions of helping that the first gel-forming layer 12a is in contact with water. In other words, the gel-forming agent contained in the first gel-forming layer 12a can be rapidly gelatinized by the sugars within the oral cavity. For these reasons, by containing the sugars in the first antiadhesive layer 13a, the edible film 1 exhibits more excellent swallowability.

Furthermore, the first antiadhesive layer 13a may contain any components other than the components described above. Examples of such components include a plasticizer, a masking agent, an antiseptic agent, a coloring agent and the like as described above.

Furthermore, a mass of the first antiadhesive layer 13a per unit area thereof is preferably in the range of 3 to 20 g/m² and more preferably in the range of 5 to 18 g/m². This makes it possible to allow an aqueous solution containing a component (antiadhesive agent) which has flowed out from the first antiadhesive layer 13a to unevenly exist around the edible film 1 for a long period of time, while providing efficiently a thin first antiadhesive layer 13a. Therefore, the edible film 1 is reliably prevented from adhering to the inside wall of the oral cavity for a longer period of time, so that it exhibits superior swallowability.

The edible film 1 as described above can be produced according to, e.g., the following processes.

### (Antiadhesive Layer Production Step)

Prepared first is a coating solution (namely, a coating solution for the antiadhesive layer) containing constituent materials of the first antiadhesive layer 13a.

The coating solution for the antiadhesive layer can be prepared by dispersing or dissolving the constituent materials of the first antiadhesive layer 13a as described above in a water medium such as purified water, ethanol or the like.

Next, the coating solution for the antiadhesive layer is applied or sprayed on a supporting substrate and then dried. This produces an antiadhesive layer to become the first antiadhesive layer 13a. In this regard, it is to be noted that an antiadhesive layer to become the second antiadhesive layer 13b can be also formed in the same manner as the process of producing the antiadhesive layer to become the first antiadhesive layer 13a.

As the supporting substrate, it is possible to use, e.g., a glass plate, a plastic film or a release sheet, but is not limited to them.

### (Gel-forming Layer production Step)

Prepared next is a coating solution (namely, a coating solution for the gel-forming layer) containing constituent materials of the first gel-forming layer 12a.

The coating solution for the gel-forming layer can be prepared by dispersing or dissolving the constituent materials of the first gel-forming layer 12a as described above in a water medium such as purified water, ethanol or the like.

Next, the coating solution for the gel-forming layer is applied or sprayed on the antiadhesive layer produced in the antiadhesive layer production step and then dried. This produces a gel-forming layer to become the first gel-forming layer 12a on the antiadhesive layer. In this regard, it is to be noted that a gel-forming layer to become the second gel-forming layer 12b can be also formed in the same manner as the process of producing the gel-forming layer to become the first gel-forming layer 12a.

### (Intermediate Body Production Step)

Prepared next is a coating solution (namely, a coating solution for the medicine-containing layer) containing constituent materials of the medicine-containing layer 11.

The coating solution for the medicine-containing layer can be prepared by dispersing or dissolving the constituent materials of the medicine-containing layer 11 as described above in a water medium such as purified water, ethanol or the like.

Next, the coating solution for the medicine-containing layer is applied or sprayed on each of the gel-forming layers produced in the gel-forming layer production step and then dried. This produces a precursor of the medicine-containing layer 11 (namely, a precursor for the medicine-containing layer) and eventually produces two intermediate bodies for the edible film (hereinafter simply referred to as an intermediate body) consisting of the precursor for the medicine-containing layer, the gel-forming layer and the antiadhesive layer.

### (Thermal Compression Bonding Step)

Next, the two intermediate bodies produced in the intermediate body production step are thermally fusion-bonded together under a pressure so that the precursors of the medicine-containing layers of the intermediate bodies can be bonded to each other. Thus, the precursors of the two medicine-containing layers are fusion-bonded to form a single medicine-containing layer. This produces an edible film 1 which is constituted from a laminated body consisting of the first and second antiadhesive layers 13a and 13b of two layers, the first and second gel-forming layers 12a and 12b of two layers and the medicine-containing layer 11. The laminated body may be used as the edible film 1 as it stands, or may be processed by a method of punching it into an arbitrary shape, such as a circular shape, an elliptical shape or a polygonal shape, to produce the edible film 1.

Furthermore, the edible film 1 may be produced by, e.g., repeating the tasks of applying and drying the coating solution for the antiadhesive layer, the coating solution for the gel-forming layer and the coating solution for the medicine-containing layer as described above.

### <Second Embodiment>

FIG. 2 is a section view showing an edible film in accordance with a second embodiment.

Hereinafter, the second embodiment of the present invention will now be described with reference to FIG. 2. The following description will be centered on the points differing from the first embodiment, with the same items omitted from the description.

As shown in FIG. 2, the edible film 1a of the present embodiment differs from that of the first embodiment in that major surfaces of first and second antiadhesive layers 13c and 13d defining outer surfaces of the edible film 1a have a plurality of convex portions 131.

Provision of the convex portions 131 on the major surfaces (outer surfaces of a laminated body) of the first and second antiadhesive layers 13c and 13d makes it possible to greatly increase a speed at which the first and second antiadhesive layers 13c and 13d absorb water from the saliva within the oral cavity. In other words, a contact area between the saliva and each of the first and second antiadhesive layers 13c and 13d can be increased by forming the convex portions 131, which results in a sharp increase in a solution rate of the first and second antiadhesive layers 13c and 13d. In addition, a contact area between an outer surface of the edible film 1a and the inside wall of the oral cavity can be reduced by forming the convex portions 131 on the major surfaces of the first and second antiadhesive layers 13c and 13d. This surely prevents the edible film 1a from adhering to the inside wall of the oral cavity. Thanks to the features noted above, the edible film 1a exhibits especially high swallowability.

The pitch P between the convex portions 131 of the first and second antiadhesive layers 13c and 13d is not particularly limited but may be preferably in the range of 100 to 1000 µm and more preferably in the range of 250 to 750 µm. This surely prevents the edible film 1a from adhering to the inside wall of the oral cavity, while rapidly dissolving the first and second antiadhesive layers 13c and 13d.

The width w of each of the convex portions 131 of the first and second antiadhesive layers 13c and 13d is not particularly limited but may be preferably in the range of 20 to 300 µm and more preferably in the range of 50 to 250 µm. This surely prevents the edible film 1a from adhering to the inside wall of the oral cavity, while rapidly dissolving the first and second antiadhesive layers 13c and 13d.

Furthermore, the height d of each of the convex portions 131 of the first and second antiadhesive layers 13c and 13d is not particularly limited but may be preferably in the range of 10 to 5000 µm and more preferably in the range of 20 to 1000 µm. This surely prevents the edible film 1a from adhering to the inside wall of the oral cavity, while rapidly dissolving the first and second antiadhesive layers 13c and 13d. Furthermore, when the edible film 1a is swallowed, it is possible to allow an aqueous solution of antiadhesive agents derived from the first and second antiadhesive layers 13c and 13d which have dissolved by water contained in saliva etc. to unevenly exist around the edible film 1 for a longer period of time. Therefore, the edible film 1 is reliably prevented from adhering to the inside wall of the oral cavity for a longer period of time.

Alternatively, a plurality of concave portions 132 of the first and second antiadhesive layers 13c and 13d may extend through the thickness of each of the first and second antiadhesive layers 13c and 13d or through the full thickness of the edible film 1a (the first and second antiadhesive layers 13c and 13d, the first and second gel-forming layers 12a and 12b and the medicine-containing layer 11).

The first and second antiadhesive layers 13c and 13d having the convex portions 131 as set forth above can be produced by, e.g., forming, on a surface of a supporting substrate, the convex portions 131 and concave portions having a pattern complementary to that of the convex portions 131 to be formed, applying the coating solution containing the constituent materials of the first and second antiadhesive layers 13c and 13d on the supporting substrate and drying the coating solution, when antiadhesive layers are produced. Alternatively, the first and second antiadhesive layers 13c and 13d having the convex portions may be produced by, e.g., producing the edible film 1 in the same manner as used in the first embodiment described above and then pressing a substrate, which has the convex portions and concave portions of a pattern complementary to that of the convex portions to be formed, against the edible film 1.

While the illustrated embodiments of the present invention have been described hereinabove, the present invention shall not be limited thereto. For example, the edible film according to the present invention is not limited to the one constituted from the first and second antiadhesive layers of the two layers, the first and second gel-forming layers of the two layers and the medicine-containing layer. For example, the edible film according to the present invention may not have the first and second gel-forming layers. Furthermore, for example, the edible film according to the present invention may have only one layer of the first and second antiadhesive layers.

Furthermore, for example, the edible film may have arbitrary layers among respective layers. For example, the edible film may have an adhesive layer for enhancing cohesion between the medicine-containing layer and each of the first and second gel-forming layers, an elution-preventing layer for preventing medicines from eluting and the like.

Furthermore, for example, a part or a whole of a lateral of the medicine-containing layer may be covered by other layers containing no medicine, e.g., the first and second gel-forming layers or the elution-preventing layer.

Furthermore, for example, the edible film may have two medicine-containing layers. This makes it possible to provide, e.g., the medicine-containing layers containing two kinds of different medicines.
Furthermore, for example, the edible film may not have the medicine-containing layer.

In this case, for example, the edible film may be a mouth freshener film which has a flavor component-containing layer containing flavor components. Examples of the flavor components include: acidic-taste imparting agents such as citric acid, tartaric acid, fumaric acid and the like; sweetening agents such as saccharin, glycyrrhizinic acid, sugars and the like; mouth fresheners such as menthol, mentha oil, peppermint, spearmint and the like; natural or synthetic perfumes; and essential oils. One or more among these compounds can be used independently or in combination.

### Examples

Next, concrete examples of the edible film according to the present invention will be described.

### 1. Production of Edible Film

### (Example 1)

### (a) Antiadhesive layers Production Step

First, a coating solution A containing constituent materials of a first antiadhesive layer was prepared.

Polyvinyl alcohol (Gosenol EG05 produced by Nippon Kasei Chemical Co., Ltd.) as an antiadhesive agent was slowly added to purified water while stirring the same. Thereafter, the purified water to which polyvinyl alcohol had added was heated to 70°C and stirred for one hour to obtain the coating solution A.

Next, the coating solution A was sufficiently defoamed. Then, the coating solution A was flat-applied on an opposite surface of a release treatment surface of a supporting substrate by using an applicator in which gaps between polyethylene terephthalate film as the supporting substrate (SP-PET3811 produced by Lintec Corp.) and a blade which the applicator had were adjusted so that an amount (mass per unit area) of an antiadhesive layer obtained after the coating solution A was dried became 15 g/m². Thereafter, the coating solution A thus applied was dried at 85°C for five minutes, thus producing an antiadhesive layer to become a first antiadhesive layer. In this regard, an antiadhesive layer to become a second antiadhesive layer was obtained by the same process as that of producing the antiadhesive layer to become the first antiadhesive layer.

### (b) Gel-forming Layers Production Step

Next, a coating solution B containing constituent materials of a first gel-forming layer was prepared.

0.60 mass parts of calcium chloride (calcium chloride defined in Japanese Pharmacopoeia and produced by Tomita Pharmaceutical Co., Ltd.) was added to 615 mass parts of purified water. The resultant mixture was stirred sufficiently to dissolve calcium chloride. As a result, an aqueous solution of calcium chloride was obtained. Then, 22.8 mass parts of polyacrylic acid (Carbopol 974P produced by CBC Co., Ltd., a viscosity of an aqueous solution of 0.2 mass% is 12100 mPa·s) was slowly added to the aqueous solution of calcium chloride while stirring the same to obtain a mixture a. After the addition of polyacrylic acid, the mixture a was stirred for about one hour. Next, 68.5 mass parts of polyvinyl alcohol (Gosenol EG05 produced by Nippon Kasei Chemical Co., Ltd.) was slowly added to the mixture a while stirring the same to obtain a mixture b. After the addition of polyvinyl alcohol, the mixture to which the respective materials had added was heated to 70°C and stirred for about one hour. Next, 8.1 mass parts of glycerin (thick glycerin defined in Japanese Pharmacopoeia and produced by ADEKA Corp.) as a water absorption promoter was added to the mixture b and stirred for about ten minutes, thereby producing a coating solution B.

Next, the coating solution B was sufficiently defoamed. Then, the coating solution B was flat-applied on the antiadhesive layer produced in the step (a) by using an applicator in which gaps between the antiadhesive layer produced in the step (a) and a blade which the applicator had were adjusted so that an amount of the antiadhesive layer after the coating solution B was dried became 20 g/m². Thereafter, the coating solution B thus applied was dried at 80°C for six minutes, thus producing a gel-forming layer to become a first gel-forming layer and obtaining a laminated body a consisting of the gel-forming layer, the antiadhesive layer and supporting substrate. In this regard, a gel-forming layer to become a second gel-forming layer was obtained by the same process as that of producing the gel-forming layer to become the first gel-forming layer, thereby obtaining a laminated body b consisting of the gel-forming layer, the antiadhesive layer and supporting substrate.

### (c) Intermediate Body Production Step

First, a coating solution C containing constituent materials of a medicine-containing layer was prepared.
2.5 mass parts of famotidine as a gastric ulcer medicine and 0.6 mass parts of titanium oxide (TIPAQUE CR-50 produced by Ishihara Sangyo Kaisha, Ltd.) were added to 53.7 mass parts of purified water and sufficiently dispersed through the use of a homogenizer to obtain dispersion liquid. Thereafter, 13.8 mass parts of polyvinyl pyrolidone (PVP K-90 produced by ISP Japan Ltd.) was slowly added to the dispersion liquid while stirring the same to obtain a mixture. After the addition of polyvinyl pyrolidone, the mixture was stirred for about thirty minutes. Next, 4.0 mass parts of glycerin (thick glycerin defined in Japanese Pharmacopoeia and produced by ADEKA Corp.) was added to the mixture and stirred for about five minutes, thereby producing a coating solution C.

Next, the coating solution C was sufficiently defoamed. Then, the coating solution C was flat-applied on each of the gel-forming layers of the laminated bodies a and b by using an applicator in which gaps between each of the gel-forming layers of the laminated bodies a and b produced in the step (b) and a blade which the applicator had were adjusted so that an amount of the medicine-containing layer after the coating solution C was dried became 50 g/m². Thereafter, the coating solution C thus applied was dried at 80°C for five minutes, thus producing a medicine-containing layer precursor to become a medicine-containing layer. Consequently, obtained were a laminated body (intermediate body a) consisting of the medicine-containing layer precursor, the gel-forming layer, the antiadhesive layer and supporting substrate and a laminated body (intermediate body b) consisting of the medicine-containing layer precursor, the gel-forming layer, the antiadhesive layer and supporting substrate.

### (d) Thermal Compression Bonding Step

The intermediate bodies a and b produced in the step (c) were thermally fusion-bonded together at a temperature of 100°C, under the conditions of a pressure of 1 kgf/cm² and for one second so that the medicine-containing layer precursors could be bonded to each other. Next, the supporting substrate was peeled off from each anthiadhesive layer, thereby producing a laminated body (edible film) that consists of two antiadhesive layers, two gel-forming layers and one medicine-containing layer. The laminated body was processed so that a shape thereof was a circular shape having a diameter of 15 mm. Consequently, produced was a circular-shaped edible film (composition: the first antiadhesive layer/ the first gel-forming layer/ the medicine-containing layer/ the second gel-forming layer/the second antiadhesive layer) which is an orally film formulation provided with a medicine-containing layer containing medicines.

### (Examples 2 to 5)

In each of the Examples 2 to 5, a kind of antiadhesive agent included in the coating solution A was changed as shown in Table 1. Furthermore, the amount of the antiadhesive agent included in the coating solution A was changed so that the amount of the antiadhesive agent included in the antiadhesive layer became the amounts as shown in Table 1. Polyvinyl alcohol (Gosenol EG05 produced by Nippon Kasei Chemical Co., Ltd.) as the antiadhesive agent was slowly added to purified water while stirring the same to obtain a mixture. Then, the mixture was heated to 70°C and stirred for one hour. Thereafter, constituent materials (antiadhesive agent) of the antiadhesive layer other than polyvinyl alcohol (antiadhesive agent) as shown in Table 1 was slowly added to the mixture, which was stirred for one hour to obtain a coating solution A. Thereafter, an edible film was produced in the same manner as in the Example 1.

### (Examples 6 to 10)

In each of the Examples 6 to 10, a kind of antiadhesive agent included in the coating solution A was changed as shown in Table 1. The changed antiadhesive agent was added to purified water, which was stirred for one hour to obtain a coating solution A. Thereafter, an edible film was produced in the same manner as in the Example 1.

### (Examples 11 and 12)

In each of the Examples 11 and 12, an edible film was produced in the same manner as in the Example 1, except that the amount of the antiadhesive layer (mass per unit area) obtained by drying the coating solution A was changed as shown in Table 1.

### (Example 13)

An edible film was produced in the same manner as in the Example 1, except that the application conditions of the coating solution A (namely, the production conditions of the antiadhesive layer) were changed as mentioned below.

First, the coating solution A was sufficiently defoamed. Then, the coating solution A was flat-applied on a polyethylene terephthalate film (supporting substrate), which had concave portions (having the mouth size of concave portions of 450x450 µm, the depth of 30 µm and the bottom size of 184x184 µm) provided in a grid pattern at a pitch of 550 µm, by using an applicator in which gaps between the polyethylene terephthalate film as the supporting substrate and a blade which the applicator had were adjusted so that an amount of the antiadhesive layer obtained by drying the coating solution A became 15 g/m². Thereafter, the coating solution A thus applied was dried at 85°C for five minutes, thus producing an adhesive layer to become a first antiadhesive layer. The antiadhesive layer thus produced was provided with convex portions having the height of about 30 µm, the width of about 450 µm and the pitch of about 550 µm, the shape of which was transferred from the concave portions. In this regard, an antiadhesive layer to become a second antiadhesive layer was obtained by the same process as that of producing the antiadhesive layer to become the first antiadhesive layer.

### (Example 14)

An edible film, which was a mouth freshener film comprised of a laminated body including an antiadhesive layer, a flavor component-containing layer and an antiadhesive layer, was produced in the same manner as in the Example 1, except that the first and second gel-forming layers were not produced and the flavor component-containing layer was produced instead of the medicine-containing layer. In this regard, the flavor component-containing layer was formed in the same process as that of forming the medicine-containing layer in the Example 1 by using a coating solution C as described below. Such a coating solution C was prepared in the same manner as in the Example 1, except that 2.5 mass parts of menthol was used in place of the 2.5 mass parts of famotidine as the gastric ulcer medicine, and a mix solvent of 34.0 mass parts of ethanol and 10.3 mass parts of purified water was used in place of the 53.7 mass parts of the purified water.

### (Comparative Example)

An edible film (composition: a first gel-forming layer, a medicine-containing layer and a second gel-forming layer) was produced in the same manner as in the Example 1, except that the antiadhesives layers were not produced.

Constituent materials and contents of the antiadhesive layer of the edible film obtained in each of the Examples and Comparative Example were shown in Table 1. In this regard, in Table 1, it is to be noted that "PVA" denotes polyvinyl alcohol (Gosenol EG05 produced by Nippon Kasei Chemical Co., Ltd., mass-average molecular weight: 30000), "PEG1" denotes polyethyelene glycol #4000 (Macrogol #4000 produced by NOF CORPORATION, mass-average molecular weight: 4000), "PEG2" denotes polyethyelene glycol #20000 (produced by Merck Ltd., mass-average molecular weight: 20000), "HPC1" denotes hydroxypropylcellulose (HPC(SL) produced by NIPPON SODA CO., LTD., mass-average molecular weight: 60000) and "MeC" denotes methylcellulose (Metolose SM-15 produced by Shin-Etsu Chemical Co., Ltd., mass-average molecular weight: 70000). Furthermore, the viscosity of the aqueous solution of 5 mass% denotes a viscosity at 37°C of an aqueous solution of 5 mass% of the constituent material (antiadhesive agent) of the antiadhesive layer. Each viscosity was measured with an E-type viscometer (a product of Tokimec, Inc.) under the conditions that a rotating speed was in the range of 10 to 100 rpm/min.

### Table 1

**Table 1**

| | Antiadhesive layer | | | | | | | Evaluation of adhesive property |
|---|---|---|---|---|---|---|---|---|
| | Antiadhesive agent | | | | | | Mass per unit area [g / m²] | |
| | Kind | Content [Mass parts] | Viscosity or aqueous solution of 5 mass% [mPa·s] | Kind | Content [Mass parts] | Viscosity of aqueous solution of 5 mass% [mPa·s] | | |
| Ex. 1 | PVA | 100 | 5.4 | - | - | - | 15 | 4.4 |
| Ex. 2 | PVA | 75 | 5.4 | Mannitol | 25 | 0.87 | 15 | 5.0 |
| Ex. 3 | PVA | 75 | 5.4 | P E G 1 | 25 | 1.29 | 15 | 5.0 |
| Ex. 4 | P V A | 75 | 5.4 | P E G 2 | 25 | 3.21 | 15 | 5.0 |
| Ex. 5 | P V A | 60 | 5.4 | M e C | 40 | 156 | 15 | 3.2 |
| Ex. 6 | M e C | 100 | 156 | - | - | - | 15 | 3.0 |
| Ex. 7 | P E G 1 | 100 | 1.29 | - | - | - | 16 | 5.0 |
| Ex. 8 | P E G 2 | 100 | 3.21 | - | - | - | 15 | 5.0 |
| Ex. 9 | H P C 1 | 100 | 16.2 | - | - | - | 15 | 4.8 |
| Ex. 10 | H P C 2 | 100 | 27.6 | - | - | - | 15 | 4.8 |
| Ex. 11 | P V A | 100 | 5.4 | - | - | - | 20 | 4.8 |
| Ex. 12 | P V A | 100 | 5.4 | - | - | - | 5 | 4.0 |
| Ex. 13 | P V A | 100 | 5.4 | - | - | - | 15 | 5.0 |
| Ex. 14 | P V A | 100 | 5.4 | - | - | - | 15 | 4.4 |
| Comp. Ex. | - | - | - | - | -- | - | - | 2.4 |

### 2. Evaluation of Adhesive Property

Gargling was conducted to cleanse the interior of the oral cavity. After two minutes, the edible film produced in each of the Examples and the Comparative Example was swallowed without water so as to on purpose adhere to the palate with ease. Thereafter, it was confirmed whether or not each edible film adhered to the palate. In the case where each edible film adhered to the palate, it was confirmed whether or not the edible film could be peeled off from the palate by tongue. The results were evaluated according to the below five criteria. In this regard, it is to be noted that the evaluation of the adhesive property of the edible film obtained in each of the Examples and the Comparative Example was carried out five times to obtain five values. Then, an average value was obtained from the obtained five values as an overall evaluation.

1······A whole of one surface of the edible film adhered to the palate, and the adhered whole of the one surface could not be peeled off by tongue with ease.
2······A part of one surface of the edible film adhered to the palate, and the adhered part of the one surface could not be peeled off by tongue with ease.
3······Although a part or whole of one surface of the edible film adhered to the palate, the adhered part or whole of the one surface could be peeled off by tongue with ease and then the edible film could be swallowed.
4······Although a part or whole of one surface of the edible film adhered to the palate, the adhered part or whole of the one surface could be quickly peeled off and then the edible film could be swallowed.
5......The edible film did not adhere to the palate, and the edible film could be swallowed smoothly.

### These results are shown together in Table 1.

As shown in Table 1, it found that it was difficult for the edible film of each Example to adhere to the inside wall of the oral cavity, and therefore it was possible to easily swallow it without water. In contrast, in the Comparative Example, no satisfactory results were obtained. In other words, the edible film of the Comparative Example adhered to the palate and there was a difficulty with swallowing it.

### EXPLANATION OF REFERENCE NUMERAL

1, 1a···edible film
11···medicine-containing layer
12a···first gel-forming layer
12b···second gel-forming layer
13a, 13c···first antiadhesive layer
13b, 13d···second antiadhesive layer
131···convex portions
132···concave portions

## Claims

1. An edible film to be ingested,
the edible film comprised of a laminated body consisting of a plurality of layers, wherein the laminated body has surfaces and the plurality of layers include two surface layers forming the surfaces of the laminated body, the plurality of layers comprising:
a medicine-containing layer containing a medicine and having one surface and an other surface opposite the one surface;
a first gel-forming layer provided on the one surface of the medicine-containing layer, and the first gel-forming layer having a first surface opposite the medicine-containing layer;
a second gel-forming layer provided on the other surface of the medicine-containing layer, and the second gel-forming layer having a second surface opposite the medicine-containing layer;
a first antiadhesive layer provided on the first surface of the first gel-forming layer; and
a second antiadhesive layer provided on the second surface of the second gel-forming layer,
wherein the two surface layers are disposed as the first and second antiadhesive layers which have a function of preventing the edible film from adhering to an inside wall of the oral cavity by dissolving with water of saliva within the oral cavity,
wherein the first antiadhesive layer contains an antiadhesive agent having a content of 90 mass% or higher, wherein when an aqueous solution of 5 mass% of the antiadhesive agent is prepared, a viscosity of the aqueous solution at 37°C is 50 mPa·s or less, and
wherein the first antiadhesive layer contains a water-soluble polymer material of (i) polyvinyl alcohol, (ii) polyvinyl alcohol and mannitol, (iii) polyvinyl alcohol and polyethylene glycol, (iv) polyvinyl alcohol and methyl cellulose, (v) methyl cellulose, (vi) polyethylene glycol, or (vii) hydroxypropylcellulose as the antiadhesive agent.

2. The edible film as claimed in claim 1, wherein a surface of each the first and second antiadhesive layer is formed with irregularities.

3. The edible film as claimed in claim 1, wherein the first gel-forming layer contains water absorption promoter for promoting a water absorption of the first gel-forming layer.

4. The edible film as claimed in claim 3, wherein the water absorption promoter includes glycerin.

5. The edible film as claimed in claim 1, wherein a mass per unit area of the first antiadhesive layer is in the range of 3 to 20 g/m².

6. The edible film as claimed in claim 1, wherein the second gel-forming layer has substantially the same configuration as the first gel-forming layer.

7. The edible film as claimed in claim 1, wherein the second antiadhesive layer has substantially the same configuration as the first antiadhesive layer.

## Patentansprüche

1. Verzehrbarer Film zur Einnahme, wobei der verzehrbare Film aus einem laminierten Körper, bestehend aus einer Vielzahl von Schichten, besteht, wobei der laminierte Körper Oberflächen aufweist und die Vielzahl von Schichten zwei Oberflächenschichten, die die Oberflächen des laminierten Körpers bilden, umfasst, wobei die Vielzahl von Schichten umfasst:
eine Medikament-enthaltende Schicht, die ein Medikament enthält und eine Oberfläche und eine andere Oberfläche gegenüber der einen Oberfläche, aufweist;
eine erste Gel-bildende Schicht, die auf der einen Oberfläche der Medikament-enthaltenden Schicht angeordnet ist, und die erste Gel-bildende Schicht eine erste Oberfläche gegenüber der Medikament-enthaltenden Schicht aufweist;
eine zweite Gel-bildende Schicht, die auf der anderen Oberfläche der Medikament-enthaltenden Schicht angeordnet ist, und die zweite Gel-bildende Schicht eine zweite Oberfläche gegenüber der Medikament-enthaltenden Schicht aufweist;
eine erste Antihaftschicht, die auf der ersten Oberfläche der ersten Gel-bildenden Schicht angeordnet ist; und
eine zweite Antihaftschicht, die auf der zweiten Oberfläche der zweiten Gel-bildenden Schicht angeordnet ist,
wobei die beiden Oberflächenschichten als die erste und die zweite Antihaftschicht angeordnet sind,
die eine Funktion haben, das Anhaften des verzehrbaren Films an eine Innenwand der Mundhöhle durch Auflösen mit Wasser der Speichelflüssigkeit innerhalb der Mundhöhle zu verhindern,
wobei die ersten Antihaftschicht ein Antihaftmittel in einem Gehalt von 90 Masse-% oder mehr enthält, wobei, wenn eine wässrige Lösung mit 5 Masse-% des Antihaftmittels hergestellt wird, die Viskosität der wässrigen Lösung bei 37 °C 50 mPa.s oder weniger beträgt, und
wobei die erste Antihaftschicht ein wasserlösliches Polymermaterial von (i) Polyvinylalkohol, (ii) Polyvinylalkohol und Mannitol, (iii) Polyvinylalkohol und Polyethylenglycol, (iv) Polyvinylalkohol und Methylcellulose, (v) Methylcellulose, (vi) Polyethylenglycol oder (vii) Hydroxypropylcellulose als das Antihaftmittel enthält.

2. Verzehrbarer Film gemäß Anspruch 1, wobei eine Oberfläche von jeder der ersten und der zweiten Antihaftschicht mit Unregelmäßigkeiten gebildet ist.

3. Verzehrbarer Film gemäß Anspruch 1, wobei die erste Gel-bildende Schicht einen Wasserabsorptionsförderer zum Fördern der Wasserabsorption der ersten Gel-Bildenden Schicht enthält.

4. Verzehrbarer Film gemäß Anspruch 3, wobei der Wasserabsorptionsförderer Glycerin umfasst.

5. Verzehrbarer Film gemäß Anspruch 1, wobei die Masse pro Flächeneinheit der ersten Antihaftschicht im Bereich von 3 bis 20 g/m² liegt.

6. Verzehrbarer Film gemäß Anspruch 1, wobei die zweite Gel-bildende Schicht im Wesentlichen den gleichen Aufbau wie die erste Gel-bildende Schicht aufweist.

7. Verzehrbarer Film gemäß Anspruch 1, wobei die zweite Antihaftschicht im Wesentlichen den gleichen Aufbau wie die erste Antihaftschicht aufweist.

## Revendications

1. Film comestible destiné à être ingéré, le film comestible étant constitué d'un corps stratifié composé d'une pluralité de couches, dans lequel le corps stratifié présente des surfaces et la pluralité de couches comprend deux couches de surfaces formant les surfaces du corps stratifié, la pluralité de couches comprenant :
une couche contenant un médicament qui contient un médicament et ayant une surface et une autre surface opposée à ladite une surface ;
une première couche formant un gel prévue sur ladite une surface de la couche contenant un médicament, et la première couche formant un gel ayant une première surface opposée à la couche contenant un médicament ;
une seconde couche formant un gel prévue sur l'autre surface de la couche contenant un médicament, et la seconde couche formant un gel ayant une seconde surface opposée à la couche contenant un médicament;
une première couche antiadhésive prévue sur la première surface de la première couche formant un gel ; et
une seconde couche antiadhésive prévue sur la seconde surface de la seconde couche formant un gel,
dans lequel les deux couches de surface sont disposées en tant que lesdites première et seconde couches antiadhésives qui ont pour fonction d'empêcher le film comestible d'adhérer à une paroi interne de la cavité buccale en se dissolvant avec de l'eau ou de la salive à l'intérieur de la cavité buccale,
dans lequel la première couche antiadhésive contient un agent antiadhésif présent en une teneur de 90% en poids ou plus, dans lequel lorsqu'une solution aqueuse de 5% en poids de l'agent antiadhésif est préparé, une viscosité de la solution aqueuse à 37°C est de 50 mPa.s ou moins, et
dans lequel la première couche antiadhésive contient un matériau polymère soluble dans l'eau composé de : (i) alcool polyvinylique, (ii) alcool polyvinylique et mannitol, (iii) alcool polyvinylique et glycol polyéthylénique, (iv) alcool polyvinylique et méthylcellulose, (v) méthylcellulose, (vi) glycol polyéthylénique, ou (vii) hydroxypropyl cellulose en tant qu'agent antiadhésif.

2. Film comestible selon la revendication 1, dans lequel une surface de chacune des première et deuxième couches antiadhésives est formée avec des irrégularités.

3. Film comestible selon la revendication 1, dans lequel la première couche formant un gel contient un promoteur de l'absorption de l'eau pour promouvoir une absorption de l'eau de la première couche formant un gel.

4. Film comestible selon la revendication 3, dans lequel le promoteur de l'absorption de l'eau comprend de la glycérine.

5. Film comestible selon la revendication 1, dans lequel une masse par unité de surface de la première couche antiadhésive est comprise dans la plage allant de 3 à 20 g/m².

6. Film comestible selon la revendication 1, dans lequel la seconde couche formant un gel a sensiblement la même configuration que la première couche formant un gel.

7. Film comestible selon la revendication 1, dans lequel la seconde couche antiadhésive a sensiblement la même configuration que la première couche antiadhésive.
